# EUROPEAN PATENT SPECIFICATION

(11) **EP 4 140 524 B1**
(45) Date of publication and mention of the grant of the patent: **04.06.2025**
(21) Application number: 21195865.7
(22) Date of filing: 09.09.2021
(51) Int. Cl.: A61M 15/00

(54) **AUTOMATIC DETECTION TRIGGER DEVICE FOR QUANTITATIVE COMPRESSED ADMINISTRATION AEROSOL**
AUTOMATISCHE DETEKTIONSAUSLÖSEVORRICHTUNG FÜR QUANTITATIV KOMPRIMIERTES VERABREICHUNGSAEROSOL
DISPOSITIF DE DÉCLENCHEMENT DE DÉTECTION AUTOMATIQUE D'UN AÉROSOL À ADMINISTRATION COMPRIMÉE QUANTITATIVE

(30) Priority: 27.08.2021 CN 202110992451
(43) Date of publication of application: 01.03.2023
(73) Proprietor: Shanghai Sonmol Internet Technology Co., Ltd., Shanghai 200050 (CN)
(72) Inventor: YE, ZhengHao, Yezhuang, 457515 (CN); ZHANG, JiGuang, Xiangyang, 441002 (CN); YANG, Ting, Beijing, 100032 (CN); NIU, HongTao, Beijing, 100089 (CN); HUANG, Ke, Beijing, 100020 (CN); JIN, JianMei, Shanghai, 201900 (CN)
(74) Representative: Ipey

(56) References cited:
- WO-A1-95/07723
- WO-A2-2017/015303
- US-A- 5 394 866
- US-A1- 2020 046 916
- US-A1- 2020 147 328

## Description

### TECHNICAL FIELD

The present invention relates to the technical field of an automatic detection trigger device, and in particular to an automatic detection trigger device for a quantitative compressed administration aerosol.

### BACKGROUND

Inhalation aerosol refers to a preparation as follows: a medicine-containing solution, suspension or emulsion, and an appropriate propellant or a liquefied mixed propellant are jointly packaged in a pressure-resistant container with a quantitative valve system and a certain pressure; and during use, the content is sprayed out as mist by means of the pressure of the propellant, and then is inhaled through a mouth and deposited in a lung. Generally, the inhalation aerosol is also called a pressure quantitative inhalant. Active substances can be quantitatively released by pressing the valve, and the medicine is dispersed into particles or frog drops which are inhaled through the respiratory tract to play a local or systemic treatment role.

The pressure quantitative inhalant, which came out in the mid-1950s, is currently the most widely used administration device. It has the advantages of small size, portability, rapid action, convenience in use, low price, multi-dose administration and the like. The structure consists of two parts: a plastic fixed base (including a suction port) and a quantitative valve. When the medicine liquid is driven by a user to manually lift and press the valve, the medicine and the propellant are sprayed out in the form of aerosol. Due to the unique design structure, the pressure quantitative aerosol inhaler can ensure that the amount of solution ejected by manual pressing is within a certain range. In order to achieve the best inhalation treatment effect, the pressure quantitative inhalant requires the user to slowly inhale deeply while manually lifting and pressing so as to inhale the medicine mist into the respiratory tract and lung. However, it is difficult for some people to ensure hand-mouth synchronization, resulting in increased deposition amount of the medicine mist in the oropharynx, low deposition rate in lung and low medicine utilization rate.

A PCT publication No. 2017015303 discloses an electronically controlled, motor-driven, breath actuated metered dose inhaler for delivering aerosolized medicament or other matter to a user. The inhaler may include a base housing including a motor-driven actuator and other system components and a removable aerosol cartridge insertably receivable in the base housing. The inhaler may be paired with a smart phone or other client computing device to provide additional functionality, such as to provide instructional information and feedback regarding usage of the inhaler, to generate and display dosage tracking information, and to provide alerts and reminders to a user of the inhaler or others.

A US application No. 2020046916 discloses a refill assembly for use in a medicinal inhaler, the refill assembly being configured to be removably coupled to a reusable assembly of a medicinal inhaler, the refill assembly comprising: a patient port; a canister actuable by the reusable assembly to deliver a dose of medicament to the patient port, a sleeve which is selectively actuable by a user independently of the reusable assembly so as to act on the canister to deliver a dose of medicament, the sleeve having a first position in which the sleeve cannot be actuated to cause a dose of medicament to be released from the canister and a second position in which the sleeve is actuable to cause a dose of medicament to be delivered to the patient port; the refill assembly further comprising: an override element which engages the sleeve, the override element being moveable from a first position in which the sleeve is retained in its first position by the override element and a second position in which the sleeve is permitted to move to its second position upon depression of the sleeve by a user.

A US patent No. 5394866 discloses a portable, battery powered, hand-held system for releasing a controlled dose of aerosol medication for inhalation by a patient including a durable body and a medication cassette inserted in the durable body. The cassette includes a housing for containing a canister of medication, bears an identification code, and permits the canister to be manually depressed to release a dose, e.g., a metered dose, when out of the durable body. The durable body includes an actuator mechanism for engaging an inserted cassette and its canister, and an actuator release mechanism for controlling the actuator mechanism to depress the canister for a selected period of time to release the desired dose of medication and then the release the canister. The actuator mechanism, includes a compression spring for depressing the canister and a torsion spring for reloading the compression spring. The torsion spring is reloaded by rotating the cassette from an open position for delivering aerosol to a closed position. The actuator release mechanism includes a motor and trigger pin assembly that controls the release of the compression spring and the torsion spring, and, hence, the time that the canister is depressed.

### SUMMARY

### (I) Technical problem to be solved

In view of the shortcomings of the prior art, the present invention provides an automatic detection trigger device for a quantitative compressed administration aerosol, so as to solve the problems in the background art.

### (II) Technical solutions

The invention is set out in the appended set of claims. In order to solve the above objective, the present invention provides the following technical solution: an automatic detection trigger device for a quantitative compressed administration aerosol includes a main machine body, wherein the main machine body consists of a main machine front cover, a main machine rear cover, a battery module and a driving module; the driving assembly includes a driving control module and a driving gear module;
the driving control module includes a driving module bracket, a display screen, a control button, a control mainboard, a driving motor, a trigger detection button, a trigger key baffle, an air inlet hole, an aerosol holder, a flow sensor and a reset spring;
the driving gear module includes a small helical gear, a first speed-reducing gear, a second speed-reducing gear, a rotary cam, a push plate and a push column;
a charging port is formed at an outer side of the main machine front cover; a limiting pedestal is clamped at a bottom end of the main machine front cover; and an aerosol medicine is fixedly mounted at the bottom of an inner cavity of the main machine front cover.

Preferably, the main machine rear cover is clamped on one side of the main machine front cover, the battery module is clamped at the main machine front cover and located below the main machine rear cover, and the driving assembly is fixedly mounted at the top of the inner cavity of the main machine front cover.

Preferably, the display screen is fixedly mounted on one side of a front surface of the driving module bracket, the control button is fixedly mounted on the front surface of the driving module bracket and located below the display screen, the control mainboard is fixedly connected to one side of the driving module bracket, and the driving motor is fixedly mounted on one side of the driving module bracket and located below the control mainboard.

Preferably, the trigger detection button is fixedly mounted at the top of the other side of the driving module bracket, the trigger key baffle is mounted below the trigger detection button, the air inlet hole is formed at the bottom of the other side of the driving module bracket, the aerosol holder is fixedly connected to a bottom end of the driving module bracket, the flow sensor is fixedly mounted on one side of an inner wall of the aerosol holder, and the reset spring is fixedly connected to a top end of the aerosol holder.

Preferably, the small helical gear is in fixed sleeving connection with an outer side of an output shaft of the driving motor, the first speed-reducing gear is engaged with a top end of the small helical gear, the second speed-reducing gear is engaged with one side of the first speed-reducing gear, the rotary camp is fixedly connected to one side of the second speed-reducing gear, the push plate is fixedly connected to a bottom end of the rotary cam, and the push column is fixedly connected to a bottom end of the push plate.

Preferably, a bottom end of the push column penetrates through the aerosol holder and is attached to a top end of the aerosol medicine, the reset spring is in sleeving connection with an outer side of the push column, and a top end of the reset spring is fixedly connected to the bottom end of the push plate.

### (III) Beneficial effects

Compared with the prior art, the present invention provides an automatic detection trigger device for a quantitative compressed administration aerosol, and has the following beneficial effects:
the automatic detection trigger device for the quantitative compressed administration aerosol may detect an inspiration flow rate of a user in a medicine inhaling process through a flow sensor arranged in the driving assembly. When the inspiration flow rate reaches an ideal threshold, the device drives the aerosol to spray a medicine, and the medicine sprayed by the aerosol enters a lower respiratory tract such as a lung along with airflow, so that the problem of hand-mouth synchronous operation is solved, and the utilization rate of the medicine is greatly increased.

### DESCRIPTION OF THE DRAWINGS

FIG. 1 is a structural schematic diagram of the present invention;
FIG. 2 is a structural schematic diagram after assembling of the present Invention;
FIG. 3 is a side view after assembling of the present Invention;
FIG. 4 is a structural schematic diagram of a driving assembly according to the present invention;
FIG. 5 is a bottom view of a driving assembly according to the present invention;
FIG. 6 is a structural schematic diagram of a driving gear module according to the present invention;
FIG. 7 is a bottom view of a driving gear module according to the present invention; and
FIG. 8 is an electrical working diagram of the present invention.

In the drawings: 1. Main machine body; 101. Main machine front cover; 102. Main machine rear cover; 2. Charging port; 3. Limiting pedestal; 4. Battery module; 5. Aerosol medicine; 67. Driving assembly; 61. Driving control module; 6101. Driving module bracket; 6102. Display screen; 6103. Control button; 6104. Control mainboard; 6105. Driving motor; 6106. Trigger detection button; 6107. Trigger key baffle; 6108. Air inlet hole; 6109. Aerosol holder; 6110. Flow sensor; 6111. Reset spring; 71. Driving gear module; 7101. Small helical gear; 7102. First speed-reducing gear; 7103. Second speed-reducing gear; 7104. Rotary camp; 7105. push plate; 7106. Push column.

### DETAILED DESCRIPTION OF EMBODIMENTS

The following clearly and completely describes the technical solutions in the embodiments of the present invention with reference to accompanying drawings in the embodiments of the present invention. Apparently, the described embodiments are merely a part rather than all of the embodiments of the present invention. All other embodiments obtained by a person of ordinary skill in the art based on the embodiments of the present invention without creative efforts shall fall within the protection scope of the present invention.

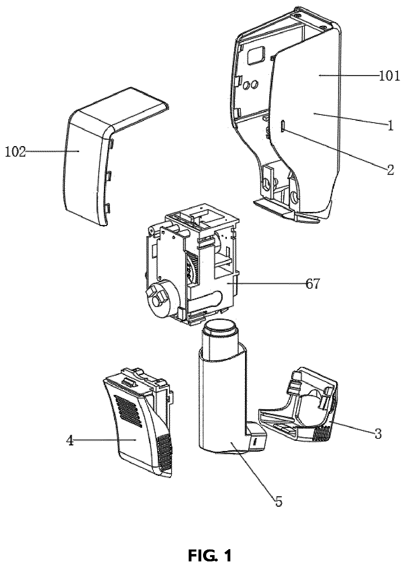

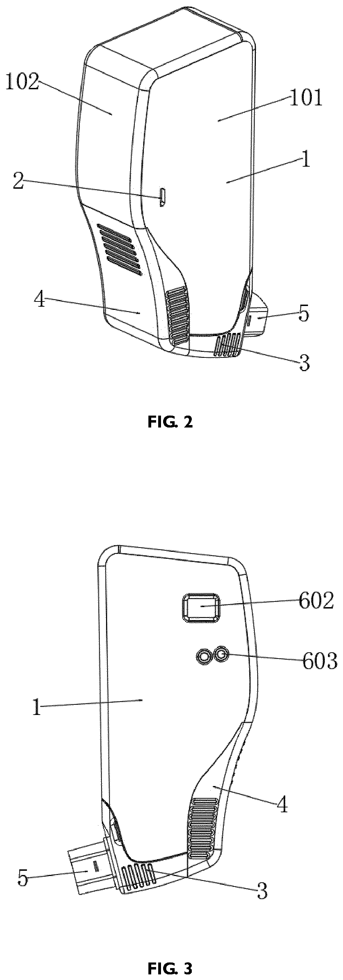

The present invention provides a technical solution: an automatic detection trigger device for a quantitative compressed administration aerosol includes a main machine body 1. Referring to FIG. 1, the main machine body 1 consists of a main machine front cover 101, a main machine rear cover 102, a battery module 4 and a driving assembly 67. Referring to FIG. 2 and FIG. 3, the main machine rear cover 102 is clamped on one side of the main machine front cover 101, the battery module 4 is clamped at the main machine front cover 101 and located below the main machine rear cover 102, the battery module 4 is located at a rear end of the main machine and is used to supply electric energy required for operation of the device, the battery module 4 adopts a chargeable lithium battery, the driving assembly 67 is fixedly mounted at the top of an inner cavity of the main machine front cover 101, a charging port 2 is formed on an outer side of the main machine front cover 101, the charging port 2 is applied to an external USB cable to charge device, a limiting pedestal 3 is clamped at the bottom end of the main machine front cover 101, an aerosol medicine 5 is fixedly mounted at the bottom of the inner cavity of the main machine front cover 101, a medicine detection sensor is mounted outside the aerosol medicine 5, and the medicine detection sensor is used to detect whether the aerosol medicine is inserted in the device. After the aerosol medicine 5 is inserted, the medicine detection sensor may detect a medicine tank so as to start a corresponding function. The common medicines in the market are stored in the aerosol medicine 5. The limiting pedestal 3 is located at the bottom of the main machine body 1 and is used to fix the aerosol medicine 5.

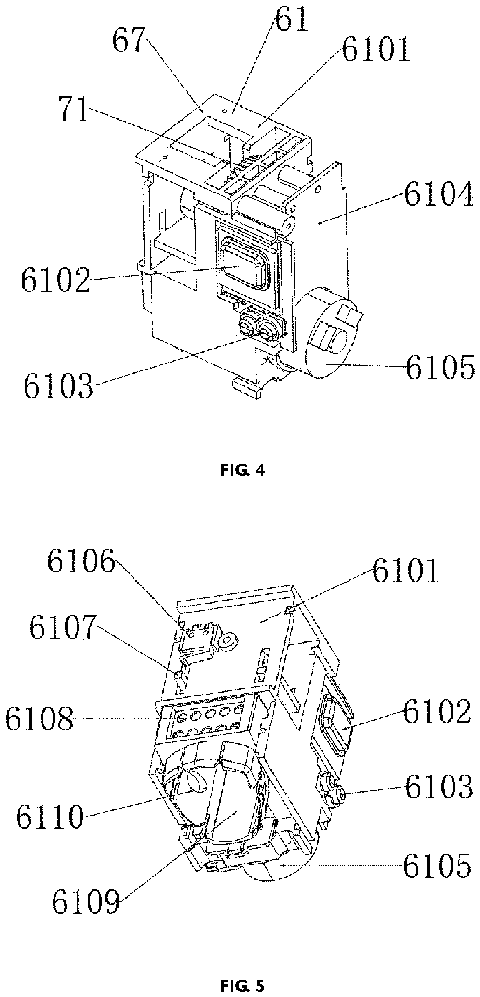

Referring to FIG. 4 and FIG. 5, the driving assembly 67 includes a driving control module 61 and a driving gear module 71, wherein the driving control module 61 includes a driving module bracket 6101, a display screen 6102, a control button 6103, a control mainboard 6104, a driving motor 6105, a trigger detection button 6106, a trigger key baffle 6107, an air inlet hole 6108, an aerosol holder 6109, a flow sensor 6110 and a reset spring 6111; and the driving module bracket 6101 is used to drive the gear module 71 to be fixed with other parts and is connected into a whole body through all the parts.

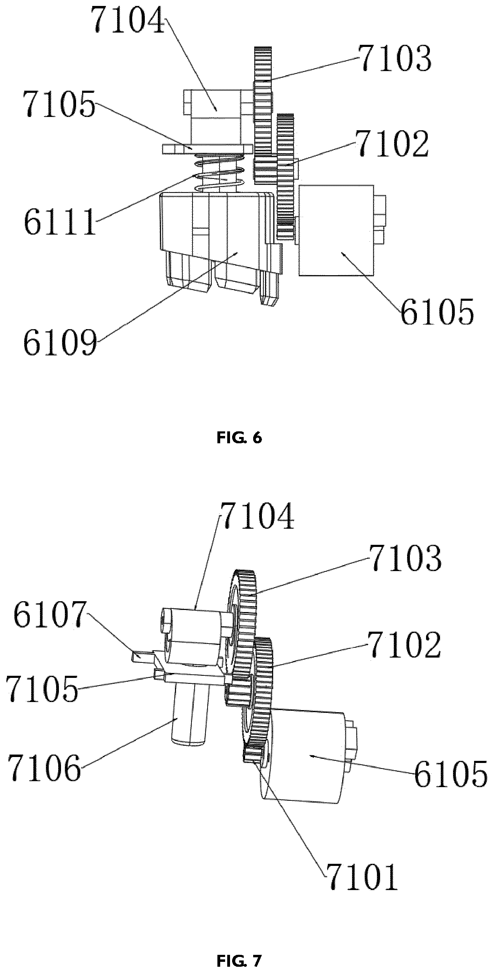

The display screen 6102 is fixedly mounted on one side of a front surface of the driving module bracket 6101, and the display screen 6102 is used to display device state information and medicine suction detection data; a user may intuitively view related information through the screen; the control button 6103 is fixedly mounted on the front surface of the driving module bracket 6101 and located below the display screen 6102, and the control button 6103 is used to control and set functions of the device; the control mainboard 6104 is fixedly connected to one side of the driving module bracket 6101, and the control mainboard 6104 is used to control various functions of the device; the driving motor 6105 is fixedly mounted on one side of the driving module bracket 6101 and located below the control mainboard 6104, and the driving motor 6105 is electrically connected to the battery module 4 through an external power line; the trigger detection button 6106 is fixedly mounted at the top of the other side of the driving module bracket 6101; the trigger key baffle 6107 is mounted below the trigger detection button 6106; the air inlet hole 6108 is formed at the bottom of the other side of the driving module bracket 6101; the aerosol holder 6109 is fixedly connected to the bottom end of the driving module bracket 6101, and the aerosol holder 6109 is located at the lower end of the driving gear module 71 and used to position the aerosol medicine 5 and the device; the flow sensor 6110 is fixedly mounted on one side of an inner wall of the aerosol holder 6109, and the flow sensor 6110 is used to detect whether the aerosol medicine 5 is inserted into the device; after the aerosol medicine 5 is inserted, the flow sensor 6110 may detect a medicine tank so as to start a corresponding function; the reset spring 6111 is fixedly connected to the top end of the aerosol holder 6109; and referring to FIG. 6, at this time, the reset spring 6111 is in an unstressed state.

Referring to FIG. 6 and FIG. 7, the driving gear module 71 includes a small helical gear 7101, a first speed-reducing gear 7102, a second speed-reducing gear 7103, a rotary cam 7104, a push plate 7105 and a push column 7106; the driving gear module 71 is a moment conduction device and performs step-by-step transfer and phase change on the moment of the driving motor 6105; the small helical gear 7101 is in fixed sleeving connection with an outer side of an output shaft of the driving motor 6105; the first speed-reducing gear 7102 is engaged with the top end of the small helical gear 7101; the second speed-reducing gear 7103 is engaged with one side of the first speed-reducing gear 7102; the rotary cam 7104 is fixedly connected to one side of the second speed-reducing gear 7103; the push plate 7105 is fixedly connected to the bottom end of the rotary cam 7104; the push column 7106 is fixedly connected to the bottom end of the push plate 7105; the bottom end of the push column 7106 penetrates through the aerosol holder 6109 and is attached to the top end of the aerosol medicine 5; the reset spring 6111 is in sleeving connection with an outer side of the push column 7106; and the top end of the reset spring 6111 is fixedly connected to the bottom end of the push plate 7105.

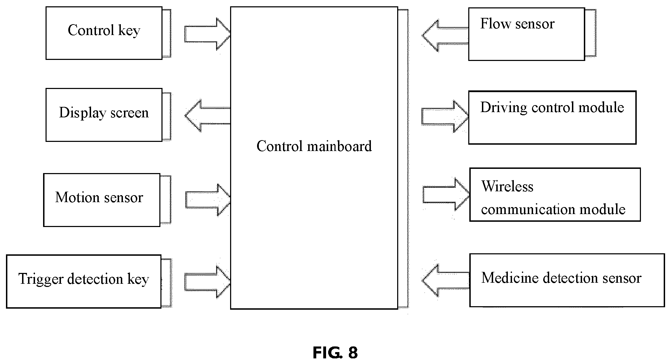

Referring to FIG. 8, when the medicine detection sensor detects that the medicine is filled in the device, the device is activated and starts to work. A motion sensor is used to detect the action and posture of medicines. Some medicines need to be shaken uniformly before use and need to be used in an erected posture. The motion sensor may detect the data so as remind the user of some unqualified use methods. The flow sensor 6110 is used to detect the flow of the inhaled gas. The control mainboard 6104 can trigger the driving control module 61 only when the detected flow continuously reaches an effective inhalation rate. A proper flow rate has a great influence on the deposition rate of the medicine, and too high or too low deposition rate will affect the absorption efficiency of the medicine. The flow sensor 6110 may detect a complete inhalation process and analyze whether the inhalation duration and the inhalation strength are reasonable, thereby giving more effective inhalation suggestions, increasing the inhalation rate of the medicine and achieving a better treatment effect.

The trigger detection button 6106 is used to detect the state of the driving control module 61 during work and feed a signal back to the control mainboard 6104. The control button 6103 is used to control information entry and function of the device, for example, resetting medicine dosage and turning pages to view data. A wireless communication module is a long-distance communication mode of the device, and the control mainboard 6104 acquires and records the device state information and the use process information and may transmit the information to a mobile phone, a computer and a cloud through a wireless communication mode, thereby facilitating data view and data analysis. The wireless communication module may be a communication mode such as Bluetooth, WIFI, a mobile information system and other wireless communication technologies.

The working principle of the device is as follows: during use, the driving motor 6105 rotates to drive the small helical gear 7101 to rotate, the small helical gear 7101 is engaged with the first speed-reducing gear 7102, the first speed-reducing gear 7102 is engaged with the second speed-reducing gear 7103, the rotary cam 7104 is fixedly on the second speed-reducing gear 7103 and rotates with the second speed-reducing gear, and a rotary moment of the driving motor 6105 is increased step by step after passing through the first speed-reducing gear 7102 and the second speed-reducing gear 7103, so that a force capable of driving the aerosol to spray the medicine is formed.

The rotary cam 7104 rotates to push the push plate 7105, the trigger key baffle 6107 is arranged on the push plate 7105, the push column 7106 is arranged below the push plate 7105, the push column 7106 is in contact with the top of the aerosol medicine 5, and the push plate 7105 moves up and down to push the push column 7106 to move up and down so as to push the aerosol medicine 5 for pressing and spraying.

When a user inhales the medicine and the control mainboard 6104 detects that the flow reaches to a proper flow, the driving assembly 67 is controlled to work, the push column 7106 pushes the medicine to complete one-time spraying, and the medicine spray enters the lower respiratory tract of a human body along with inhalation. After the push column 7106 pushes the medicine, the trigger key baffle 6107 triggers the trigger detection button 6106 and feeds a feedback signal back to the control mainboard 6104, and the control mainboard 6104 controls the driving assembly 67 to stop work, so that the medicine is automatically detected for one time.

It should be noted that in this specification, relational terms such as first and second are used only to differentiate an entity or operation from another entity or operation, and do not require or imply that any actual relation or sequence exists between these entities or operations. In addition, the terms "comprise", "include" and any other variants thereof are intended to cover non-exclusive inclusion, so that a process, a method, an article, or a device that includes a series of elements not only includes these very elements, but may also include other elements not expressly listed, or also include elements inherent to this process, method, article, or device.
Although the embodiments of the present invention have been illustrated and described, it should be understood that those of ordinary skill in the art may make various changes, modifications, replacements and variations to the above embodiments without departing from the principle of the present invention, and the scope of the present invention is limited by the appended claims

## Claims

1. An automatic detection trigger device for a quantitative compressed administration aerosol, comprising a main machine body (1), wherein the main machine body (1) consists of a main machine front cover (101), a main machine rear cover (102), a battery module (4) and a driving assembly (67); the driving assembly (67) comprises a driving control module (61) and a driving gear module (71);
the driving control module (61) comprises a driving module bracket (6101), a control mainboard (6104) configured to control the driving assembly (67) to work or stop work, a driving motor (6105), an air inlet hole (6108), an aerosol holder (6109), a flow sensor (6110) and a reset spring (6111); the control mainboard (6104) is fixedly connected to one side of the driving module bracket (6101), and the driving motor (6105) is fixedly mounted on one side of the driving module bracket (6101) and located below the control mainboard (6104);
the driving gear module (71) comprises a small helical gear (7101), a first speed-reducing gear (7102), a second speed-reducing gear (7103), a rotary cam (7104), a push plate (7105) and a push column (7106); the push column (7106) is fixedly connected to a bottom end of the push plate (7105);
the reset spring (6111) is fixedly connected to a top end of the aerosol holder (6109); the reset spring (6111) is in sleeving connection with an outer side of the push column (7106), and a top end of the reset spring (6111) is fixedly connected to the bottom end of the push plate (7105);
a charging port (2) is formed at an outer side of the main machine front cover (101); a limiting pedestal (3) is clamped at a bottom end of the main machine front cover (101); and an aerosol medicine (5) is fixedly mounted at a bottom of an inner cavity of the main machine front cover (101); the limiting pedestal (3) is configured to fix the aerosol medicine (5);
the driving motor (6105) rotates to drive the small helical gear (7101) to rotate, the small helical gear (7101) is engaged with the first speed-reducing gear (7102), the first speed-reducing gear (7102) is engaged with the second speed-reducing gear (7103), the rotary cam (7104) is fixedly on the second speed-reducing gear (7103) and rotates with the second speed-reducing gear (7103); the rotary cam (7104) rotates to push the push plate (7105); the push column (7106) is in contact with a top end of the aerosol medicine (5), and the push plate (7105) moves up and down to push the push column (7106) to move up and down, such that the push column (7106) pushes the aerosol medicine (5) for pressing and spraying;
the flow sensor (6110) is configured to detect a flow of an inhaled gas; **characterized in that**
a medicine detection sensor is mounted outside the aerosol medicine (5); the medicine detection sensor is configured to detect whether the aerosol medicine (5) is inserted in the automatic detection trigger device;
the driving control module (61) further comprises a trigger detection button (6106), a trigger key baffle (6107); the trigger detection button (6106) is fixedly mounted at a top of an other side of the driving module bracket (6101) to the control mainboard (6104) or the driving motor (6105);
the trigger key baffle (6107) is arranged on the push plate (7105) and mounted below the trigger detection button (6106); after the push column (7106) pushes the aerosol medicine (5), the trigger key baffle (6107) triggers the trigger detection button (6106), then the trigger detection button (6106) feeds a feedback signal back to the control mainboard (6104), and the control mainboard (6104) controls the driving assembly (67) to stop work.

2. The automatic detection trigger device for the quantitative compressed administration aerosol according to claim 1, **characterized in that** the main machine rear cover (102) is clamped on one side of the main machine front cover (101), the battery module (4) is clamped at the main machine front cover (101) and located below the main machine rear cover (102), and the driving assembly (67) is fixedly mounted at a top of the inner cavity of the main machine front cover (101).

3. The automatic detection trigger device for the quantitative compressed administration aerosol according to claim 1, **characterized in that** the driving control module (61) further comprises a display screen (6102) and a control button (6103); the display screen (6102) is fixedly mounted on one side of a front surface of the driving module bracket (6101), the control button (6103) is fixedly mounted on the front surface of the driving module bracket (6101) and located below the display screen (6102); the display screen (6102) is configured to display device state information and medicine suction detection data; the control button (6103) is configured to control and set functions of the automatic detection trigger device.

4. The automatic detection trigger device for the quantitative compressed administration aerosol according to claim 1, **characterized in that** the air inlet hole (6108) is formed at a bottom of the other side of the driving module bracket (6101), the aerosol holder (6109) is fixedly connected to a bottom end of the driving module bracket (6101), the flow sensor (6110) is fixedly mounted on one side of an inner wall of the aerosol holder (6109).

5. The automatic detection trigger device for the quantitative compressed administration aerosol according to claim 1, **characterized in that** the small helical gear (7101) is in fixed sleeving connection with an outer side of an output shaft of the driving motor (6105), the first speed-reducing gear (7102) is engaged with a top end of the small helical gear (7101), the second speed-reducing gear (7103) is engaged with one side of the first speed-reducing gear (7102), the rotary cam (7104) is fixedly connected to one side of the second speed-reducing gear (7103).

6. The automatic detection trigger device for the quantitative compressed administration aerosol according to claim 1, **characterized in that** a bottom end of the push column (7106) penetrates through the aerosol holder (6109) and is attached to the top end of the aerosol medicine (5).

7. The automatic detection trigger device for the quantitative compressed administration aerosol according to claim 1, wherein the charging port (2) is configured to charge the automatic detection trigger device through an external USB cable.

## Patentansprüche

1. Eine automatische Erkennungsauslösevorrichtung für ein quantitatives komprimiertes Verabreichungsaerosol, umfassend: einen Hauptmaschinenkörper (1); wobei der Hauptmaschinenkörper (1) aus einer Hauptmaschinen-Frontabdeckung (101), einer Hauptmaschinen-Rückabdeckung (102), einem Batteriemodul (4), und einer Antriebsbaugruppe (67) besteht; die Antriebsbaugruppe (67) ein Antriebssteuermodul (61) und ein Antriebsgetriebemodul (71) umfasst;
das Antriebssteuermodul (61) eine Antriebsmodulhalterung (6101), eine Steuerungshauptplatine (6104), die so konfiguriert ist, dass sie die Antriebsbaugruppe (67) zum Arbeiten oder zum Stoppen der Arbeit steuert, einen Antriebsmotor (6105), eine Lufteinlassöffnung (6108), einen Aerosolhalter (6109), einen Durchflusssensor (6110), und eine Rückstellfeder (6111) umfasst; die Steuerungshauptplatine (6104) fest mit einer Seite der Antriebsmodulhalterung (6101) verbunden ist und der Antriebsmotor (6105) fest an einer Seite der Antriebsmodulhalterung (6101) angebracht und unterhalb der Steuerungshauptplatine (6104) angeordnet ist;
das Antriebsgetriebemodul (71) ein kleines Schrägverzahnungsrad (7101), ein erstes Untersetzungszahnrad (7102), ein zweites Untersetzungszahnrad (7103), einen Drehnocken (7104), eine Druckplatte (7105), und eine Drucksäule (7106) umfasst; die Drucksäule (7106) fest mit einem unteren Ende der Druckplatte (7105) verbunden ist;
die Rückstellfeder (6111) fest mit einem oberen Ende des Aerosolhalters (6109) verbunden ist; die Rückstellfeder (6111) in einer Hülsenverbindung mit einer Außenseite der Drucksäule (7106) steht, und ein oberes Ende der Rückstellfeder (6111) fest mit dem unteren Ende der Druckplatte (7105) verbunden ist;
eine Ladeöffnung (2) an einer Außenseite der Hauptmaschinen-Frontabdeckung (101) ausgebildet ist; ein Begrenzungsfuß (3) an einem unteren Ende der Hauptmaschinen-Frontabdeckung (101) festgeklemmt ist; und ein Aerosolmedikament (5) fest an einer Unterseite eines inneren Hohlraums der Hauptmaschinen-Frontabdeckung (101) angebracht ist; der Begrenzungsfuß (3) so konfiguriert ist, dass er das Aerosolmedikament (5) fixiert;
der Antriebsmotor (6105) sich dreht, um das kleine Schrägverzahnungsrad (7101) in Drehung zu versetzen, das kleine Schrägverzahnungsrad (7101) mit dem ersten Untersetzungszahnrad (7102) in Eingriff ist, das erste Untersetzungszahnrad (7102) mit dem zweiten Untersetzungszahnrad (7103) in Eingriff ist, der Drehnocken (7104) fest an dem zweiten Untersetzungszahnrad (7103) befestigt ist und sich mit dem zweiten Untersetzungszahnrad (7103) dreht; der Drehnocken (7104) sich dreht, um die Druckplatte (7105) zu drücken; die Drucksäule (7106) in Kontakt mit einem oberen Ende des Aerosolmedikaments (5) steht, und die Druckplatte (7105) sich auf und ab bewegt, um die Drucksäule (7106) auf und ab zu bewegen, so dass die Drucksäule (7106) das Aerosolmedikament (5) zum Drücken und Sprühen drückt;
der Durchflusssensor (6110) so konfiguriert ist, dass er einen Durchfluss eines inhalierten Gases erfasst; **dadurch gekennzeichnet, dass**
ein Medikamentenerkennungssensor außerhalb des Aerosolmedikaments (5) angebracht ist; der Medikamentenerkennungssensor so konfiguriert ist, dass er erkennt, ob das Aerosolmedikament (5) in die automatische Erkennungsauslösevorrichtung eingesetzt ist;
das Antriebssteuermodul (61) ferner einen Auslöseerkennungsknopf (6106) und eine Auslösetastabdeckung (6107) umfasst; der Auslöseerkennungsknopf (6106) fest an einer Oberseite einer anderen Seite der Antriebsmodulhalterung (6101) an der Steuerungshauptplatine (6104) oder dem Antriebsmotor (6105) angebracht ist; die Auslösetastabdeckung (6107) auf der Druckplatte (7105) angeordnet und unterhalb des Auslöseerkennungsknopfes (6106) angebracht ist; nachdem die Drucksäule (7106) das Aerosolmedikament (5) gedrückt hat, die Auslösetastabdeckung (6107) den Auslöseerkennungsknopf (6106) auslöst, woraufhin der Auslöseerkennungsknopf (6106) ein Rückmeldesignal an die Steuerungshauptplatine (6104) zurücksendet und die Steuerungshauptplatine (6104) die Antriebsbaugruppe (67) zum Stoppen der Arbeit ansteuert.

2. Automatische Erkennungsauslösevorrichtung nach Anspruch 1, wobei die Hauptmaschinen-Rückabdeckung (102) an einer Seite der Hauptmaschinen-Frontabdeckung (101) festgeklemmt ist, das Batteriemodul (4) an der Hauptmaschinen-Frontabdeckung (101) geklemmt und unterhalb der Hauptmaschinen-Rückabdeckung (102) angeordnet ist, und die Antriebsbaugruppe (67) fest an einer Oberseite des inneren Hohlraums der Hauptmaschinen-Frontabdeckung (101) angebracht ist.

3. Automatische Erkennungsauslösevorrichtung nach Anspruch 1, wobei das Antriebssteuermodul (61) ferner einen Bildschirm (6102) und eine Steuertaste (6103) umfasst; der Bildschirm (6102) fest an einer Seite einer Vorderseite der Antriebsmodulhalterung (6101) angebracht ist, die Steuertaste (6103) fest an der Vorderseite der Antriebsmodulhalterung (6101) angebracht und unterhalb des Bildschirms (6102) angeordnet ist; der Bildschirm (6102) so konfiguriert ist, dass er Informationen zum Gerätestatus und Daten zur Medikamentenansaugerkennung anzeigt; die Steuertaste (6103) so konfiguriert ist, dass sie Funktionen der automatischen Erkennungsauslösevorrichtung steuert und einstellt.

4. Automatische Erkennungsauslösevorrichtung nach Anspruch 1, wobei die Lufteinlassöffnung (6108) an einer Unterseite der anderen Seite der Antriebsmodulhalterung (6101) ausgebildet ist, der Aerosolhalter (6109) fest mit einem unteren Ende der Antriebsmodulhalterung (6101) verbunden ist, und der Durchflusssensor (6110) fest an einer Seite einer Innenwand des Aerosolhalters (6109) angebracht ist.

5. Automatische Erkennungsauslösevorrichtung nach Anspruch 1, wobei das kleine Schrägverzahnungsrad (7101) in fester Hülsenverbindung mit einer Außenseite einer Abtriebswelle des Antriebsmotors (6105) steht, das erste Untersetzungszahnrad (7102) mit einem oberen Ende des kleinen Schrägverzahnungsrads (7101) in Eingriff steht, das zweite Untersetzungszahnrad (7103) mit einer Seite des ersten Untersetzungszahnrads (7102) in Eingriff steht, der Drehnocken (7104) fest mit einer Seite des zweiten Untersetzungszahnrads (7103) verbunden ist.

6. Automatische Erkennungsauslösevorrichtung nach Anspruch 1, wobei ein unteres Ende der Drucksäule (7106) durch den Aerosolhalter (6109) hindurchragt und an dem oberen Ende des Aerosolmedikaments (5) befestigt ist.

7. Automatische Erkennungsauslösevorrichtung nach Anspruch 1, wobei die Ladeöffnung (2) so konfiguriert ist, dass sie die automatische Erkennungsauslösevorrichtung über ein externes USB-Kabel auflädt.

## Revendications

1. Dispositif de déclenchement de détection automatique pour un aérosol à administration comprimée quantitative, comprenant un corps de machine principale (1), dans lequel le corps de machine principale (1) se compose d'un couvercle avant de machine principale (101), d'un couvercle arrière de machine principale (102), d'un module de batterie (4) et d'un ensemble d'entraînement (67) ; l'ensemble d'entraînement (67) comprend un module de commande d'entraînement (61) et un module d'engrenage d'entraînement (71) ;
le module de commande d'entraînement (61) comprend un support de module d'entraînement (6101), une carte mère de commande (6104) conçue pour commander l'ensemble d'entraînement (67) pour fonctionner ou arrêter le fonctionnement, un moteur d'entraînement (6105), un orifice d'entrée d'air (6108), un support d'aérosol (6109), un capteur de débit (6110) et un ressort de rappel (6111) ; la carte mère de commande (6104) est reliée de manière fixe à un côté du support de module d'entraînement (6101), et le moteur d'entraînement (6105) est monté de manière fixe sur un côté du support de module d'entraînement (6101) et situé au-dessous de la carte mère de commande (6104) ;
le module d'engrenage d'entraînement (71) comprend un petit engrenage hélicoïdal (7101), un premier engrenage réducteur de vitesse (7102), un second engrenage réducteur de vitesse (7103), une came rotative (7104), une plaque de poussée (7105) et une colonne de poussée (7106) ; la colonne de poussée (7106) est reliée de manière fixe à une extrémité inférieure de la plaque de poussée (7105) ;
le ressort de rappel (6111) est relié de manière fixe à une extrémité supérieure du support d'aérosol (6109) ; le ressort de rappel (6111) est manchonné sur un côté externe de la colonne de poussée (7106), et une extrémité supérieure du ressort de rappel (6111) est reliée de manière fixe à l'extrémité inférieure de la plaque de poussée (7105) ;
un port de charge (2) est formé sur un côté externe du couvercle avant de machine principale (101) ; un socle de limitation (3) est fixé au niveau d'une extrémité inférieure du couvercle avant de machine principale (101) ; et un médicament d'aérosol (5) est monté de manière fixe au niveau d'une partie inférieure d'une cavité interne du couvercle avant de machine principale (101) ; le socle de limitation (3) est conçu pour fixer le médicament d'aérosol (5) ;
le moteur d'entraînement (6105) tourne afin d'entraîner la rotation du petit engrenage hélicoïdal (7101), le petit engrenage hélicoïdal (7101) est en prise avec le premier engrenage réducteur de vitesse (7102), le premier engrenage réducteur de vitesse (7102) est en prise avec le second engrenage réducteur de vitesse (7103), la came rotative (7104) est fixée sur le second engrenage réducteur de vitesse (7103) et tourne avec le second engrenage réducteur de vitesse (7103) ; la came rotative (7104) tourne afin de pousser la plaque de poussée (7105) ; la colonne de poussée (7106) est en contact avec une extrémité supérieure du médicament d'aérosol (5), et la plaque de poussée (7105) se déplace vers le haut et vers le bas afin de pousser la colonne de poussée (7106) à se déplacer vers le haut et vers le bas, de sorte que la colonne de poussée (7106) pousse le médicament d'aérosol (5) afin de le presser et le pulvériser ;
le capteur de débit (6110) est conçu pour détecter un débit d'un gaz inhalé ; **caractérisé en ce que**
un capteur de détection de médicament est monté à l'extérieur du médicament d'aérosol (5) ; le capteur de détection de médicament est conçu pour détecter si le médicament d'aérosol (5) est inséré dans le dispositif de déclenchement de détection automatique ;
le module de commande d'entraînement (61) comprend en outre un bouton de détection de déclenchement (6106), un déflecteur de touche de déclenchement (6107) ; le bouton de détection de déclenchement (6106) est monté de manière fixe au niveau d'une partie supérieure d'un autre côté du support de module d'entraînement (6101) par rapport à la carte mère de commande (6104) ou au moteur d'entraînement (6105) ;
le déflecteur de touche de déclenchement (6107) est disposé sur la plaque de poussée (7105) et monté au-dessous du bouton de détection de déclenchement (6106) ; après que la colonne de poussée (7106) a poussé le médicament aérosol (5), le déflecteur de touche de déclenchement (6107) déclenche le bouton de détection de déclenchement (6106), puis le bouton de détection de déclenchement (6106) renvoie un signal de rétroaction à la carte mère de commande (6104), et la carte mère de commande (6104) commande l'ensemble d'entraînement (67) pour arrêter le fonctionnement.

2. Dispositif de déclenchement de détection automatique pour l'aérosol à administration comprimée quantitative selon la revendication 1, **caractérisé en ce que** le couvercle arrière de machine principale (102) est fixé sur un côté du couvercle avant de machine principale (101), le module de batterie (4) est fixé au niveau du couvercle avant de machine principale (101) et situé au-dessous du couvercle arrière de machine principale (102), et l'ensemble d'entraînement (67) est monté de manière fixe au niveau d'une partie supérieure de la cavité interne du couvercle avant de machine principale (101).

3. Dispositif de déclenchement de détection automatique pour l'aérosol à administration comprimée quantitative selon la revendication 1, **caractérisé en ce que** le module de commande d'entraînement (61) comprend en outre un écran d'affichage (6102) et un bouton de commande (6103) ; l'écran d'affichage (6102) est monté de manière fixe sur un côté d'une surface avant du support de module d'entraînement (6101), le bouton de commande (6103) est monté de manière fixe sur la surface avant du support de module d'entraînement (6101) et situé au-dessous de l'écran d'affichage (6102) ; l'écran d'affichage (6102) est conçu pour afficher des informations d'état de dispositif et des données de détection d'aspiration de médicament ; le bouton de commande (6103) est conçu pour commander et régler des fonctions du dispositif de déclenchement de détection automatique.

4. Dispositif de déclenchement de détection automatique pour l'aérosol à administration comprimée quantitative selon la revendication 1, **caractérisé en ce que** l'orifice d'entrée d'air (6108) est formé au niveau d'une partie inférieure de l'autre côté du support de module d'entraînement (6101), le support d'aérosol (6109) est relié de manière fixe à une extrémité inférieure du support de module d'entraînement (6101), le capteur de débit (6110) est monté de manière fixe sur un côté d'une paroi interne du support d'aérosol (6109).

5. Dispositif de déclenchement de détection automatique pour l'aérosol à administration comprimée quantitative selon la revendication 1, **caractérisé en ce que** le petit engrenage hélicoïdal (7101) est manchonné de manière fixe sur un côté externe d'un arbre de sortie du moteur d'entraînement (6105), le premier engrenage réducteur de vitesse (7102) est en prise avec une extrémité supérieure du petit engrenage hélicoïdal (7101), le second engrenage réducteur de vitesse (7103) est en prise avec un côté du premier engrenage réducteur de vitesse (7102), la came rotative (7104) est reliée de manière fixe à un côté du second engrenage réducteur de vitesse (7103).

6. Dispositif de déclenchement de détection automatique pour l'aérosol à administration comprimée quantitative selon la revendication 1, **caractérisé en ce qu'**une extrémité inférieure de la colonne de poussée (7106) pénètre à travers le support d'aérosol (6109) et est attachée à l'extrémité supérieure du médicament aérosol (5).

7. Dispositif de déclenchement de détection automatique pour l'aérosol à administration comprimée quantitative selon la revendication 1, dans lequel le port de charge (2) est conçu pour charger le dispositif de déclenchement de détection automatique par l'intermédiaire d'un câble USB extérieur.
